# EUROPEAN PATENT APPLICATION

(11) **EP 2 359 877 A1**
(43) Date of publication of application: **24.08.2011**
(21) Application number: 10153306.5
(22) Date of filing: 11.02.2010
(51) Int. Cl.: A61L 27/20, A61L 27/22, A61L 27/24, A61L 27/38, A61L 27/54

(54) **Bicompatible vascularising and cicatrizing drug-delivery matrix**

(71) Applicant: Universidad de Salamanca, 37008 Salamanca (ES)
(72) Inventor: Martin del Valle, Eva María, 37008, Salamanca (ES); Galan Serrano, Miguel Angel, 37008, Salamanca (ES); Perez Herrero, Edgar, 37008, Salamanca (ES); Varela Simo, Gonzalo, 37008, Salamanca (ES); Aranda Alcaide, Jose Luís, Salamanca (ES)
(74) Representative: Pons Ariño, Angel

(57) **Abstract**

The present invention relates to a biocompatible vascularising and cicatrizing drug-delivery matrix that stimulates the vascularisation, cicatrisation and the regeneration of tissues and organs, a method to develop it, and the use to vascularised and cicatrize tissues and organs. Matrixes obtained by the method comprise at least the release of one bioactive-compound.

## Description

### OBJECT OF THE INVENTION

The main object of the present invention relates to a biocompatible drug-delivery matrix that allows the vascularization, cicatrization and the regeneration of tissues, a method to develop it, and the use to vascularize and cicatrize tissues.

Matrixes obtained by the method comprise at least the release of one bioactive-molecule.

### BACKGROUND OF THE INVENTION

Tissue engineering has been an active field of research for several decades now. However, its clinical application is still limited due to some unsolved problems, such as the poor blood supply for the allografts in the initial phase after implantation. Insufficient vascularisation can lead to improper cell integration or cell death in tissue-engineered constructs.

Sometimes it is necessary to remove an organ or part of it (e.g., the lung), due to cancer or other illnesses. After whole lung removal (what we call a pneumonectomy), one of the most feared complications is post pneumonectomy bronchopleural fistula (BPF), communication between the bronchial tree and the pleural space through the bronchial stump responsible for patient deterioration, infection and finally death *"*Cerfolio RJ. The incidence, etiology, and prevention of postresectional bronchopleural fistula. Semin Thorac Cardiovasc Surg 2001; 13: 3-7*" "*Jiménez MF, Varela G, Novoa N, Aranda JL. Sleeve lobectomy compared to pneumonectomy for the treatment of N0-N1 non-small cell lung cancer.Arch Bronconeumol 2006; 42:160-4*.* "

In those circumstances in which the bronchial stump is at risk for fistula development (ischemia, infection...), adequate healing and revascularization of the bronchial suture line is essential in order to avoid postoperative morbidity and mortality, not to mention the rising of in-hospital charges due to prolongued or repeated treatments.

Fibrin was originally developed as a sealant and an adhesive in surgery, as it is critical for natural wound healing. Fibrin gels can be prepared from the patient's own blood by the enzymatic polymerization of fibrinogen in the presence of thrombin at room temperature. Fibrin gels can be degraded and remodeled by cell associated enzymatic activity during cell migration and wound healing. Fibrin gels have been used as an autologous scaffold to engineer tissues using skeletal muscle cells, smooth muscle cells, and chondrocytes.

Several factors complicate the development of cellular therapies. Of primary importance is protection of the implanted cells from the host's immune system to prevent the freshly grafted cells from attack by native killer cells.

A highly undesirable solution to immuno-rejection is the regular administration of a cocktail of immunosuppressants that can result in serious side effects.

In order to solve the immuno-rejection problem and give mechanical protection to the graft, cells and factors have to be administered to the patient within scaffolds, may be immobilized in biocompatible supports with semi-permeable membranes that so much assures the mechanical protection as block entry of immune mediators, allowing outward diffusion of the growth factors produced by the cells. In addition, this membrane permits the entry of nutrients and oxygen, and the exit of waste *"*Benoit et al. Microencapsulation methods and industrial applications. Marcel Dekker: New York, 1996*': "*Orive G et al. Cell encapsulation: promise and progress. Nat Med 2003; 9: 104-107*", "*Angelova N, Hunkeler D. Rationalizing the design of polymeric biomaterials Trends Biotechnol 1999; 17: 409-421*": "*Orive G et al. History, challenges and promises of cell microencapsulation. Trends Biotechnol 2004; 22: 87-92*" and "*De Vos P, Hamel AF, Tatarkiewicz K Considerations for successful transplantation of encapsulated pancreatic islets. Diabetologia 2002; 45: 159-173*".*

During the last years, the major efforts have been dedicated to develop spherical microcapsules to cell therapy *"*Herrero EP, Del Valle EMM and Galan MA. Immobilization of Mesenchymal stem cells and monocytes in biocompatible microcapsules to cell therapy. Biotechnol Prog 2007; 23: 940-945" but recently other type of cell immobilization polymeric devices is taking importance, the immobilisation within polymeric films *"*Jain K et al. Retrievable, replaceable, macroencapsulated pancreatic islet xenografts. Long-term engraftment without immunosuppression. Transplantation 1995; 59:319-324*".*

Cell immobilisation within films presents some advantages in comparison with microcapsules in some kinds of treatments; they are easy to retrieval in case of complications *"*Kizilel S, Garfinkel M and Opara E. The bioartificial pancreas: progress and challenges. Diabetes technology & therapeutics 2005; 7(6): 968-985*"* and are useful in tissue repairing *"*Alperin C, Zandstra PW and VVoodhouse KA. Polyurethane films seeded with embryonic stem cell-derived cardiomyocytes for use in cardiac tissue engineering applications. Biomaterials 2005; 26: 7377-7386*"* and wounds treatment. In addition their shape, structure and mechanical strength are adequate to be fixed using sutures by surgery.

Alginate is a widely used natural polymer for tissue engineering applications due to its excellent biocompability and biodegradability. Probably, the most important property of alginates is their ability to form gels by reaction with divalent cations such as calcium or barium.

Although the biocompability, biodegradability and usefulness of alginates and fibrin gels in tissue engineering are documented *"*Lee KY. Design parameters of polymers for tissue engineering applications. Macromolecular research 2005; 13(4): 277-284*"* there is not described technology that reports a biocompatible support of the cells and factors, with the enough mechanical strength to be sutured, completely fixed to the bronchial stump to allow the revascularisation to the treatment of the BPF.

In order to solve this problem, the present invention describes the design and develops a new biocompatible drug-delivery matrix that comprises, two faces of equal or different structure: one for its adherence to the organ or tissue, and the other one for a protection of the area. Moreover these kinds of matrixes comprise growth factors that prevent the degradation and optimize the vascularisation.

### DESCRIPTION OF THE INVENTION

The present invention relates to a new kind of drug delivery matrixes to vascularize, cicatrize and regenerate tissues and/or organs. These matrixes comprise two faces with equal or different structure. The first one it is for the adherence to the damaged organ or tissue, and second one for a protection of the area.

Therefore, in a first aspect, the present invention is related to a new kind of biocompatible, vascularizing and cicatrizing drug-delivery matrixes for the administration of active compounds comprising two layers with equal or different structure.

The first one is an adherence layer that comprises non-globular proteins. These non-globular proteins are selected from the group consisting of fibrous proteins. Particularly are selected from the group consisting of fibrin, fibrinogen, collagen, or mixtures thereof.

The second one is an active protection layer that comprises one or more substances or combinations thereof selected from the group comprising polysaccharide salts and active compounds. More particularly, the salt of the polysaccharide is selected for the group consisting of alginate, hyalurinate, agar, collagen, albumin, gelatin agarose, carrageenan, or mixtures thereof. and the cation is selected from the group consisting of lithium, sodium, potassium, calcium, barium, iron, copper, magnesium, cadmium, and strontium.

Moreover, the active compounds comprise one or more substances or combinations thereof selected from the group comprising growth factors that prevent the degradation and optimize the vascularisation and adherent cells.

According to another preferred embodiment, the growth factors comprise one or more combinations thereof selected from the group comprising vascular endotelial growth factor, basic fibroblast growth factor, platelet-derived growth factor, insulin-like growth factor-1, granulocyte-colony stimulating factor, granulocyte-macrophage colony stimulating factor, epidermal growth factor, hepatocyte growth factor and erythropoietin, all of them free or in biocompatible calcium-polysaccharide microcapsules.

According to another preferred embodiment, the adherent cells are selected from stem cells, epithelial cells, fibroblasts, keratinocytes or mixtures thereof.

This new matrix allows the sequential release of one or more different molecules simultaneously. The inclusion of polysaccharide salts and active compounds stimulate the vascularization and the regeneration of the tissues and organs.

According to another preferred embodiment, these kind of biocompatible drug-delivery matrixes has a third protection layer that comprises one or more substances or combinations thereof selected from the group comprising polysaccharide salts and active compounds More particularly, the salt of the polysaccharide is selected for the group consisting of hyalurinate, alginate, chitosan, or mixtures thereof and the cation is selected from the group consisting of lithium, sodium, potassium, calcium, barium, iron, copper, magnesium, cadmium, and strontium.

Moreover, the active compounds comprise one or more substances or combinations thereof selected from the group comprising growth factors that prevent the degradation and optimize the vascularization and adherent cells.

According to another preferred embodiment, the growth factors comprise one or more combinations thereof selected from the group comprising vascular endotelial growth factor, basic fibroblast growth factor, platelet-derived growth factor, insulin-like growth factor-1, granulocyte-colony stimulating factor, granulocyte-macrophage colony stimulating factor, epidermal growth factor, hepatocyte growth factor and erythropoietin, all of them free or in biocompatible calcium-polysaccharide microcapsules.

According to another preferred embodiment, the adherent cells are selected from stem cells, epithelial cells, fibroblasts, keratinocytes or mixtures thereof.

This new third layer allows the sequential release of one or more different molecules simultaneously. The inclusion of polysaccharide salts and active compounds stimulate the vascularization and the regeneration of the tissues and organs.

The second and third layers could have the same or different composition or structure.

The expression "active compounds" or "bioactive compounds" as used herein is intended to encompass agents other than foods, which promote a structural and/or functional change in and/or on bodies to which they have been administered. They may also be an agent used in the recognition of a disease or condition. These agents are not particularly limited; however, they should be physiologically acceptable and compatible with the film.

Other examples of active compounds include anesthetics, analgesics, adherent cells, drugs for psychiatric disorders, epilepsies, growth factors, migraine, stopping drug additions and buses; antiinflammatory agents, drugs to treat hypertension, cardiovascular diseases, gastric acidity and GI ulcers; drugs for hormone replacement therapies and contraceptives; antibiotics and other antimicrobial agents; antineoplastic agents, immunosuppressive agents and immunostimulants; and drugs acting on blood and the blood forming organs including hematopoietic agents and anticoagulants, thrombolytics, and antiplatelet drugs. Other active compounds suitable for transdermal delivery to treat allergies are selected from the group consisting of fine particles or extracts from natural substances, e.g., from herbs, grass seeds, pollens, and animal debris. Other suitable active compounds include, but are not limited to 1) antimicrobial agents, such as triclosan, cetyl pyridium chloride, domiphen bromide, quaternary ammonium salts, zinc compounds, sanguinarine, fluorides, alexidine, octonidine, EDTA, and the like; 2) non-steroidal antiinflammatory drugs, such as aspirin, acetaminophen, ibuprofen, ketoprofen, diflunisal, fenoprofen calcium, naproxen, tolmetin sodium, indomethacin, and the like; 3) anti-tussives, such as benzonatate, caramiphen edisylate, menthol, dextromethorphan hydrobromide, chlophedianol hydrochloride, and the like; 4) decongestants, such as pseudoephedrine hydrochloride, phenylepherine, phenylpropanolamine, pseudoephedrine sulfate, and the like; 5) anti-histamines, such as brompheniramine maleate, chlorpheniramine maleate, carbinoxamine maleate, clemastine fumarate, dexchlorpheniramine maleate, diphenhydramine hydrochloride, diphenylpyraline hydrochloride, azatadine meleate, diphenhydramine citrate, doxylamine succinate, promethazine hydrochloride, pyrilamine maleate, tripelennamine citrate, triprolidine hydrochloride, acrivastine, loratadine, brompheniramine, dexbrompheniramine, and the like; 6) expectorants, such as guaifenesin, ipecac, potassium iodide, terpin hydrate, and the like; 7) anti-diarrheals, such a loperamide, and the like; 8) H2-antagonists, such as famotidine, ranitidine, and the like; 9) proton pump inhibitors, such as omeprazole, lansoprazole, and the like; 10) general nonselective CNS depressants, such as aliphatic alcohols, barbiturates and the like; 1 1 ) general nonselective CNS stimulants such as caffeine, nicotine, strychnine, picrotoxin, pentylenetetrazol and the like; 12) drugs that selectively modify CNS function, such as phenyhydantoin, phenobarbital, primidone, carbamazepine, ethosuximide, methsuximide, phensuximide, trimethadione, diazepam, benzodiazepines, phenacemide, pheneturide, acetazolamide, sulthiame, bromide, and the like; 13) antiparkinsonism drugs such as levodopa, amantadine and the like; 14) narcotic-analgesics such as morphine, heroin, hydromorphone, metopon, oxymorphone, levorphanol, codeine, hydrocodone, xycodone, nalorphine, naloxone, naltrexone and the like; 15) analgesic-antipyretics such as salycilates, phenylbutazone, indomethacin, phenacetin and the like; and 16) psychopharmacological drugs such as chlorpromazine, methotrimeprazine, haloperidol, clozapine, reserpine, imipramine, tranylcypromine, phenelzine, lithium and the like.

A further aspect of the present invention is a biocompatible vascularizing and cicatrizing drug-delivery matrix, as defined above, for its use to vascularize and cicatrize tissues and/or organs.

The matrixes defined above, are prepared according to the following procedure:
a) A first active compound is suspended in DI water until a final concentration ranged (1-5%) mg/mL.
b) Then, it is prepared a 1-5 % wt. polysaccharide salt solution where is suspended the first active solution, and/or at least a second active compound. This second active compound could be free or encapsulated in microcapsules preferably in calcium-polysaccharide microcapsules and it is selected from the group consisting of growth factors and adherent cells.
c) Next step comprises to extend a thin layer (1-6 mL) of the 1-5 % wt. polysaccharide solution (with the active compounds as described above) over a Petri dish with a porous filter paper soaked in a 1-6 % wt. barium chloride solution or over a two porous plates in contact with a gripper to incorporate the hardening solution.
d) After that, the layer is kept five minutes under the crosslinking conditions, leaving free the upper surface without the crosslinking solution to allow the adherence layer adhere to the protection or polysaccharide layer.
e) Finally, it is prepared the adherence layer (1-6 mL) over the free surface of polysaccharide layer as was described above.
f) Then, the biphasic film is stored in an incubator at 37 ºC and 1-5% of CO₂ for culturing.
g) After 1-5 days of culturing, the adherence layer is adhered to the polysaccharide layer because of the non-globular proteins growth, and then it is recovered with a barium chloride membrane in order to protect the matrix by means of immerse the matrix in a 1-6 % wt. barium chloride bath.

Optionally is possible to produce a triphasic matrix incorporating a second protection layer following the same steps to produce the first one. The third layer could incorporate the same or different active compounds than the second one.

Another aspect of the invention is the use of the biocompatible drug-delivery matrixes to vascularize and cicatrize tissues and/or organs of some important diseases but also industrial applications, i.e., intelligent textiles, cosmetics, etc.

In a further aspect, the present invention is related to the use of biocompatible, vascularizing and cicatrizing drug-delivery matrix for pneumonectomy brochopleural fistula prophylaxis, thoracic wounds healing and soft tissue regeneration

### DESCRIPTION OF THE DRAWINGS

In order to complement the description being made and with the purpose of helping towards a better understanding of the characteristics of the invention, in accordance with the examples, a set of drawings is attached as an integral part of said description, wherein the following, in an illustrative and non-limitative character, has been represented:
Figure 1.- Depicts a device used to prepared films with cells. (1) represent the entrance of barium chloride, (2) represents a porous plate in a glass support, (3) represents the entrance of polysaccharide solution with active compounds and (4) represents a filter.
Figure 2.- Depicts a macroscopic histological section of a control-group pig bronchial stump. There is scarce widening over the suture line (see arrows), corresponding to normal reparative fibrosis.

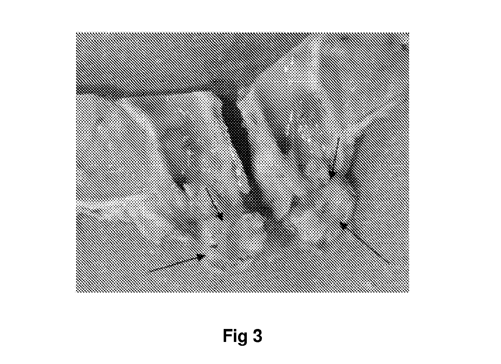

Figure 3.- Depicts a macroscopic histological section of an experimental-group pig bronchial stump. There is a significative widening over the suture line (see arrows), corresponding to intense reparative process by means of revascularized fibrous tissue.

### EXAMPLES

In view of the figures, several embodiments of the unfolding module object of this invention are described.

### Example 1 Method to the preparation of the fibrinogen film face (4 mL).

The recipe to obtain a fibrinogen gel of 4 mL of volume was the following:
- The starting solution is 1.6 mL of plasma from blood
- Add 1.840 µL of complete growth medium supplemented with 10 % fetal calf serum and 1 % penicillin/streptomycin to the plasma.
- Suspend 40 000 fibroblast with 240 µL of medium in the former mix.
- Add the stabilizing agent of the fibrinogen clot, tranexamic acid (160 µL).
- Add 160 µL of calcium chloride (final concentration 4 mg/mL).
- Mix the sample by pipetting.
- Storage of the mix in an incubator during 2 days for culturing at 37 ºC and 5% of CO₂ (Forma Direct Heat CO₂ Incubator, Thermo Electron Corporation, Hucoa-Erlöss, Model 311).
- Add enough complete growth medium to the fibrin films to avoid desecation.

All solutions used were completely sterile.

The whole process was performed under sterile conditions in a class II laminar flow hood (Nuaire, Model NO-NU-425-40DE).

### Example 2 Method to the preparation of the fibrin film face (2 mL).

The recipe to obtain a fibrin gel of 2 mL of volume was the following:
- The starting solution is 0.8 mL of plasma from blood
- Add 920 µL of complete growth medium supplemented with 10 % fetal calf serum and 1 % penicillin/streptomycin to the plasma.
- Suspend 40 000 fibroblast with 120 µL of medium in the former mix.
- Add the stabilizing agent of the fibrin clot, tranexamic acid (80 µL).
- Add 80 µL of calcium chloride (final concentration 4 mg/mL).
- Mix the sample by pipetting.
- Storage of the mix in an incubator during 2 days for culturing at 37 ºC and 5% of CO₂ (Forma Direct Heat CO₂ Incubator, Thermo Electron Corporation, Hucoa-Erlöss, Model 311).
- Add enough complete growth medium to the fibrin films to avoid desecation.

All solutions used were completely sterile.

The whole process was performed under sterile conditions in a class II laminar flow hood (Nuaire, Model NO-NU-425-40DE).

### Example 3 Technology to the preparation of the protective film face.

This process is based on the ionic gelation reaction. This method consists of the suspension of the active material that will be immobilized in a water polianion solution, which usually is a polysaccharide, i.e., sodium alginate, and then the mixture is stabilized by means of a water solution of cations, i.e., barium chloride, producing a semi-permeable membrane with the adequate characteristics in terms of pore size and mechanical strength.

This method consists in extend a thin layer of a 2 % wt. alginate solution over a Petri dish with a porous filter paper soaked in a 3 % wt. barium chloride solution and then make crosslinking adding dropwise a barium chloride solution over the upper surface, this last process was applied in this invention, but with this method is difficult to control film thickness and uniformity.

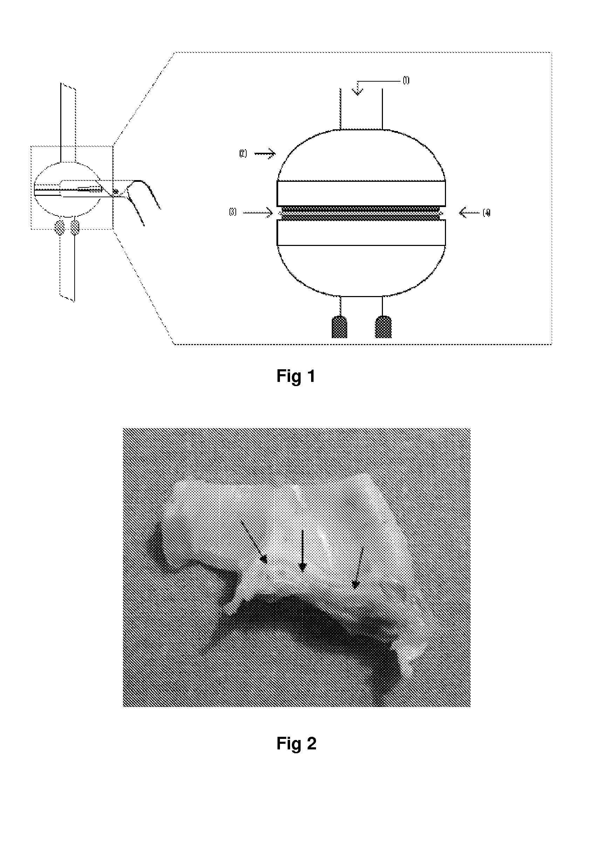

This method of production of films described before is optimized, to control and reduce the thickness (300 microns) maintaining the mechanical strength. The device to generate the biocompatible films (Figure 1) was composed of two porous plates, fixated in a metallic support, in contact with a gripper. In each part of the device was placed a filter drenched with a 3 % wt. barium chloride. On the plate that was set in the support, was spilled a 2 % wt. alginate solution, in the center of the filter. The other plate was then vertically placed over the first one, and the device was fixed with the gripper. Through the entrance on the top of the device was introduced 4 mL of barium chloride solution so that it could cross the porous plate and contacting with the alginate thus enabling the formation of the gel which constitutes the membrane.

All solutions used were autoclaved using a steam sterilizer (P-Selecta, Autester-E) at 121 ºC for 30 min. The alginate powder was sterilized by exposure to UV radiation during at least 48 hours.

All the equipment used, including all the glassware was autoclaved at 121 ºC for 30 min. The whole process was performed under sterile conditions in a class II laminar flow hood (Nuaire, Model NO-NU-425-40DE).

### Example 4 Technology to the generation of the microcapsules.

It was also used the ionic gelation technique, described before, to produce the capsules. This method adapted to the generation of capsules consists of the suspension of the active material (free bFGF) in sodium alginate, and then the mixture is stabilized by adding dropwise into the barium chloride, producing a semipermeable membrane. The microbeads produced were kept five minutes under the crosslinked conditions and a continuous agitation and, finally, collected by filtration with nylon filters.

In this case, the ionic gelation technique needs a technology that provides the drops. The technology used to the generation of the drops was an electro-magnetic (laminar jet break-up) encapsulation unit (Nisco Encapsulation Unit, Var D, LIN-0085) that has a single vibrating nozzle where frequency and amplitude can be adjusted digitally. A syringe pump produces a steady pulsation free flow through the vibrating nozzle.

### Example 5 Technology to the generation of the biphasic matrix of fibrinogen or fibrin and alginate.

The matrix was generated by means of two different procedures:

### a) Generation of the biphasic matrix by means of Petri dish:

First, the VEGF was suspended in DI water until a final concentration of 1 µg/mL. After that, it was generated the microcapsules with the bFGF incorporated as was described before. Then, it was prepared a 2 % wt. alginate solution where was suspended the VEGF solution, the microcapsules containing the bFGF, and fibroblast.

This method consists of extend a thin layer (4 mL) of the 2 % wt. alginate solution (with cells, factors and capsules) over a Petri dish with a porous filter paper soaked in a 3 % wt. barium chloride solution. After that the layer was kept five minutes under the crosslinking conditions, leaving free the upper surface without the crosslinking solution to allow the fibrin adhere to the alginate layer.

Finally, it was prepared the fibrinogen layer (4 mL) over the free surface of alginate as was described above. Then, the biphasic film was stored in an incubator at 37 ºC and 5% of CO₂ for culturing. After two days of culturing, the fibrinogen layer was adhered to the alginate layer because of the fibrinogen growth, and then it was recovered with a barium chloride membrane in order to protect the matrix by means of immerse the matrix in a 3 % wt. barium chloride bath.

### b) Generation of the biphasic matrix by means of porous plate:

First, the VEGF was suspended in DI water until a final concentration of 1 µg/mL. After that, it was generated the microcapsules with the bFGF incorporated as was described before. Then, it was prepared a 2 % wt. alginate solution where was suspended the VEGF solution, the microcapsules containing the bFGF, and fibroblast.

This method consists of two porous plates, fixated in a metallic support, in contact with a gripper. In each part of the device was placed a filter drenched with a 3 % wt. barium chloride solution, in this case, the upper filter was drenched with a 1 % wt. barium chloride solution to avoid the complete crosslinking reaction on the upper surface of the matrix. On the plate that was set in the support, was spilled the 2 % wt. alginate solution (with cells, factors and capsules) in the center of the filter (2 mL). The other plate was then vertically placed over the first one, and the device was fixed with the gripper. In this case it was avoided adding barium chloride solution throughout the entrance on the top of the device so that it does not generate the complete crosslinking process. It was produced an incomplete membrane with the enough mechanical strength to work with it but that allows the fibrin layer to adhere to the upper surface of the film.

Finally, it was prepared the fibrin layer (2 mL) over the upper surface of the film as was described above. Then, the biphasic film was stored in an incubator at 37 ºC and 5% of CO₂ for culturing. After two days of culturing, the fibrin layer was adhered to the alginate layer because of the fibrin growth, and then it was recovered with a barium chloride membrane in order to protect the matrix by means of immerse the matrix in a 3 % wt. barium chloride bath.

### Example 6 Study of efficiency of biphasic drug-delivery matrix

Post pneumonectomy bronchopleural fistula (BPF) is a very serious complication after lung surgery, due to its high mortality (33%) *"*Novoa N, Aranda JL, Jiménez MF, Varela G. Usefullness of muscle transposition for thoracostomy and postpneumonectomy bronchopleural fistula closure in a single surgical intervention. Cir. Cardiov. 2009;16(2):153-8*".* and impairment of patient quality of life, specially in those cases of infection and sepsis when the only therapeutical option is a mutillating surgery called *thoracostomy* (creation of a thoracic window by means of muscle and ribs resection for allowing infection drainage). Patients need daily attention and cures (sometimes for years) to allow infection to heal before attempting surgical closure of this "window".

Some circumstances favour the development of a BPF through interference in the normal bronchial stump healing process. Thus, infection and devascularisation result in bronchial suture necrosis and BPF. In an effort to prevent BPF occurrence in those particular high-risk cases, the development of a manufacturable, autologous and avascular allograft capable to heal and revascularize the bronchial stump would be of great interest.

These results must be considered as pilot results, based only on observational and descriptive histological techniques (hematoxiline-eosine and inmunohistochemical staining with CD-31 antibodies for vessel identification).

35 pigs wheighing 15 to 25 kilograms were allocated to different groups:
**1. Control Group (Group C)** Figure 2: pneumonectomy with closure of the bronchial stump in a standard fashion (surgical manual sutures): 5 ANIMALS
   * Poor inflammatory response
   * Fibroblastic proliferation is scarce and corresponding to a normal healing process ("reparative fibrosis")
   * No evidence for revascularization
**2. Group Simple Membrane (Group MS):** pneumonectomy with closure of the bronchial stump in a standard fashion (surgical manual sutures) plus suture line coverage with biphasic membrane (alginate plus fibrin layer): 10 ANIMALS
   * Moderate inflammatory response
   * Fibroblastic proliferation is moderate and corresponding to a normal healing process ("reparative fibrosis") + some degree of foreign body tissular response to alginate
   * Revascularization is scarce
**3. Group Complete Tissue Allograft (Group CTA):** pneumonectomy with closure of the bronchial stump in a standard fashion (surgical manual sutures) plus suture line coverage with biphasic membrane (alginate plus fibrin layer) in addition to tissue- engineered homologous fibroblasts with free vascular endothelial growing factor (VEGF) and microencapsulated fibroblast growing factor (FGF) : 20 ANIMALS
   * Intense inflammatory response (the more intense registered between the three groups)
   * Fibroblastic proliferation shoots up on postoperative day 7, then stabilizes and gradually decreases until postoperative day 30. Fibroblastic cells are fully viable and demonstrate biological activity (positive proliferation, migration...), which means that VEGF and FGF are active on them.
   * On postoperative day 7, vessel formation is demonstrated with evidence of nascent capillary vessels, continuing to complete vascular structures on postoperative day 14.

NOTE: We have only demonstrated the positive angiogenetic effect of the allograft. These neovessels must be thoroughly studied in terms of vascular density (number of mature vessels per mm3) and vessels caliber to fully assure its normal functionallity.

To resume, the application of a biocompatible vascularizing and cicatrizing drug-delivery matrix seems to be effective for BPF prophylaxis through bronchial stump revascularization and healing improvement.

## Claims

1. A biocompatible, vascularising and cicatrizing drug-delivery matrix for administration of active compounds comprising:
a. adherence layer; and
b. active protection layer.

2. Biocompatible vascularising and cicatrizing drug-delivery matrix according to claim 1, wherein the adherence layer comprise non-globular proteins.

3. Biocompatible vascularising and cicatrizing drug-delivery matrix according to claim 2, wherein the non-globular proteins comprise fibrous proteins.

4. Biocompatible vascularising and cicatrizing drug-delivery matrix according to claim 3, wherein the selected proteins are fibrin, fibrinogen, collagen or mixtures thereof.

5. Biocompatible vascularising and cicatrizing drug-delivery matrix according with anyone of claims 1 to 4, wherein the protection layer comprises one or more substances or combinations thereof selected from the group comprising polysaccharide salts and an active compounds.

6. Biocompatible vascularising and cicatrizing drug-delivery matrix according to claim 5, wherein the polysaccharide salt is selected from alginate, hyalurinate, agar, collagen, albumin, gelatin agarose, carrageenan, or mixtures thereof.

7. Biocompatible vascularising and cicatrizing drug-delivery matrix according to claim 5, wherein the active compounds comprises one or more substances or combinations thereof selected from the group comprising growth factors and adherents cells.

8. Biocompatible vascularising and cicatrizing drug-delivery matrix according to claims 7, wherein the growth factors comprise one or more substances or combinations thereof selected from the group comprising vascular endotelial growth factor, basic fibroblast growth factor, platelet-derived growth factor, insulin-like growth factor-1, granulocyte-colony stimulating factor, granulocyte-macrophage colony stimulating factor, epidermal growth factor, hepatocyte growth factor and erythropoietin, all of them free or in a biocompatible calcium-polysaccharide microcapsules.

9. Biocompatible vascularising and cicatrizing drug-delivery matrix according to claim 7, wherein the adherent cells are selected from stem cells, epithelial cells, fibroblast, keratinocytes or mixtures of those

10. Biocompatible vascularising and cicatrizing drug-delivery matrix according to anyone of claims 1 to 9, wherein the matrix comprises an additional third layer that comprises the same characteristics of the protection layer according to claims 5 to 9.

11. Biocompatible vascularising and cicatrizing drug-delivery matrix according to anyone of claims 1 to 10, for use to vascularise and cicatrise tissues and/or organs.

12. Method for the production of a biocompatible vascularising and cicatrizing drug-delivery matrix according to claims 1 to 10 **characterised by** comprising the following steps:
a. suspend in DI water a first active compound;
b. prepare a 1 or 2 solutions of polysaccharide salt;
c. suspend the first active compound and/or at least a second compound free or encapsulated in microcapsules, into the polysaccharide salt solution;
d. stabilization of the mixture (c) over a petry dish with porous filterpaper soaked of the hardening solution or over a two porous plates in contact with a gripper to incorporate the hardening solution;
e. prepare an adherence layer of non-globular proteins; and
f. Incorporation of the adherence layer over the free surface of resulting layer from (d).

13. Use of the biocompatible vascularising and cicatrizing drug-delivery matrix according to claims 1 to 10 to vascularise and cicatrise tissues and/or organs.

14. Use of the biocompatible vascularising and cicatrizing drug-delivery matrix according to claims 1 to 10, for pneumonectomy brochopleural fistula prophylaxis, thoracic wounds healing and soft tissue regeneration.
